(19) Europäisches Patentamt
European Patent Office
Office européen des brevets

(11) Publication number: 0 114 389
A2

# (12) EUROPEAN PATENT APPLICATION

(21) Application number: 83113028.1

(22) Date of filing: 23.12.83

(51) Int. Cl.³: C 07 C 49/82
C 07 C 49/84, C 07 C 49/86
C 07 C 45/28, C 07 C 45/30
C 07 C 45/64, C 07 C 143/00
C 07 C 97/10, C 07 C 101/60

(30) Priority: 20.01.83 US 459449
20.01.83 US 459450
27.01.83 US 461337

(43) Date of publication of application:
01.08.84 Bulletin 84/31

(84) Designated Contracting States:
AT BE CH DE FR GB IT LI NL SE

(71) Applicant: AMERICAN CYANAMID COMPANY
1937 West Main Street P.O. Box 60
Stamford Connecticut 06904(US)

(72) Inventor: Floyd, Middleton Brawner, Jr.
5 Babbling Brook Road
Suffern New York 10901(US)

(72) Inventor: DeVries, Vern Gordon
425 Farview Street
Ridgewood New Jersey 07450(US)

(74) Representative: Wächtershäuser, Günter, Dr.
Tal 29
D-8000 München 2(DE)

(54) **Substituted arylglyoxal derivatives.**

(57) Arylglyoxals which are new compounds and which are active as hypoglycemic agents.

EP 0 114 389 A2

This invention relates to new arylglyoxals and to their hydrates and addition products, their use in the treatment of diabetes mellitus, pharmaceutical compositions containing them and their chemical synthesis. More particularly, it is concerned with compounds which may be represented by the formula:

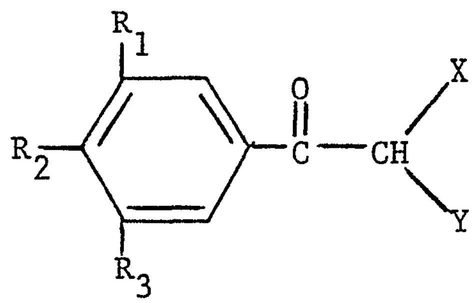

wherein $R_1$ is hydrogen, methyl or chloro; $R_2$ is hydrogen, methyl, chloro, 1-naphthyloxy, 4-chloro-1-naphthyloxy, 4-chloro-2-naphthyloxy or moieties of the formulae:

$-O-(CH_2)_n-R_4$ 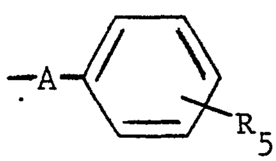

wherein n is an integer from 1 to 4 and $R_4$ is selected from the group consisting of $(C_4-C_6)$cycloalkyl, phenyl, pentafluorophenyl and monosubstituted phenyl wherein the substituent is fluoro, chloro, methyl, methoxy, trifluoromethyl or phenoxy; A is oxo (-O-), thio (-S-) or imino (-NH-) and $R_5$ represents at least one substituent selected from the group consisting of hydrogen, halogen, $(C_1-C_4)$alkylthio and trifluoromethyl; $R_3$ is hydrogen, methyl, chloro or a moiety of the formula: $-A-C_6H_4-R_5$ wherein A and $R_5$ are as hereinbefore defined; X and Y may be the same or different and are independently selected from the group consisting of hydroxy, $(C_1-C_4)$alkoxy, anilino, <u>para</u>-carboxyanilino, <u>para</u>-$(C_2-C_4)$-carboalkoxyanilino and $-SO_3Q$ wherein Q is an alkali metal or alkaline earth metal and X and Y taken together represents keto (O=); and the hydrates thereof with the first proviso that $R_1$, $R_2$ and $R_3$ may not all be hydrogen, and with the second proviso that when $R_1$ and $R_3$ are hydrogen

then $R_2$ may not be methyl or chloro, and with the third proviso that when n is 1 then $R_4$ may not be phenyl, and with the fourth proviso that when A is oxo or thio then $R_5$ may not be hydrogen or halogen , and with the fifth proviso that $R_2$ and $R_3$ may not both be the moiety $-A-C_6H_4-R_5$.

This invention is further concerned with a method of treating diabetes mellitus in a mammal in need of such treatment which comprises administering to said mammal an effective amount of a compound as recited above.

This invention also relates to a method of treating hyperglycemia in a mammal in need of such treatment which comprises administering to said mammal an effective amount of a compound as recited above.

This invention also relates to a pharmaceutical composition which comprises an effective antidiabetic amount of a compound as recited above in association with a pharmaceutically acceptable carrier.

This invention also relates to a pharmaceutical composition which comprises an effective hypoglycemic amount of a compound as recited above in association with a pharmaceutically acceptable carrier.

Finally, this invention relates to processes for preparing compounds as recited above.

Certain of the novel arylglyoxals of the present invention may be prepared according to the following reaction sequence:

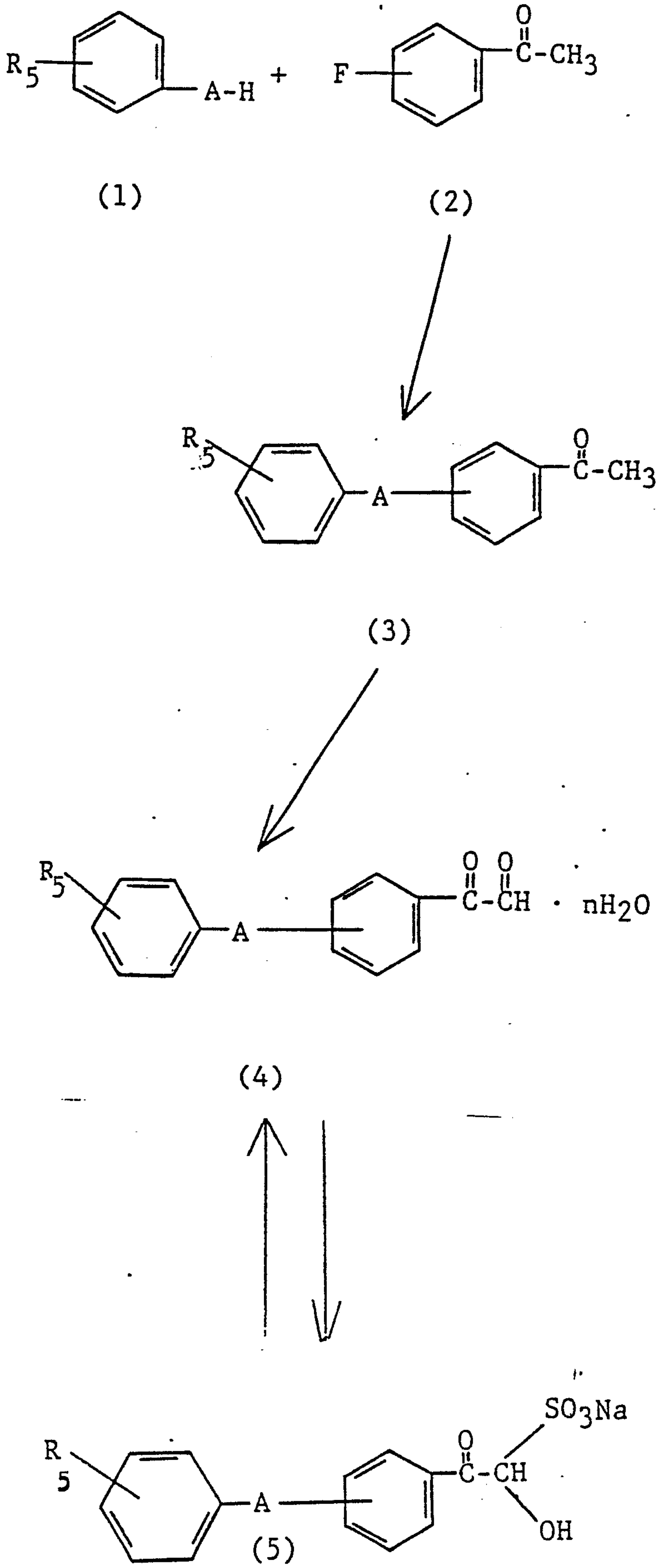

(1) (2)

(3)

(4)

(5)

In accordance with the above reaction sequence, a phenol or thiophenol (1) where $R_5$ is as described above and A is oxygen or sulfur is combined with a fluoroacetophenone (2) and treated with potassium carbonate in dimethylacetamide under an inert atmosphere at reflux for 8-48 hours, then cooled, poured into ice and extracted with ether. The extract is evaporated and distilled, giving the acetophenone (3) where $R_5$ is as described above and A is oxygen or sulfur. The acetophenone (3) is then dissolved in dimethylsulfoxide and treated with aqueous hydrobromic acid at 45-65°C. for 18-48 hours, poured into ice and extracted with ethyl acetate. The extract containing (4) where n may vary from almost zero to one or more, is concentrated, dissolved in a mixture of ethanol and water at 50-70°C. and treated with sodium metabisulfite at the boiling point for 5 minutes, then cooled under argon at 0°C., giving (5), where $R_5$ and A are as described above. The sodium bisulfite derivative (5) is then suspended in water at 40-60°C., acidified, heated at 90-100°C. for 1-2 hours, cooled and extracted with ether. The ether extract is concentrated giving (4) where $R_5$ and A are as described above and A is oxo or thio. Compounds of structure (4) are obtained in various degrees of hydration; that is, n may vary from almost zero to one or more.

---

Alternatively, the acetophenone (3), where $R_5$ and A are as described above, is treated with selenium dioxide in aqueous dioxane at reflux, under an inert atmosphere for 12-24 hours. The reaction mixture is then filtered and the filtrate evaporated, giving (4) where $R_5$ is as described above, which may be converted to the methanesulfonic acid, sodium salt derivative (5) by treatment with sodium metabisulfite in aqueous ethanol.

To produce the compounds where $R_5''$ is as described above and A is -NH-, an appropriate substituted diphenylamine (6)

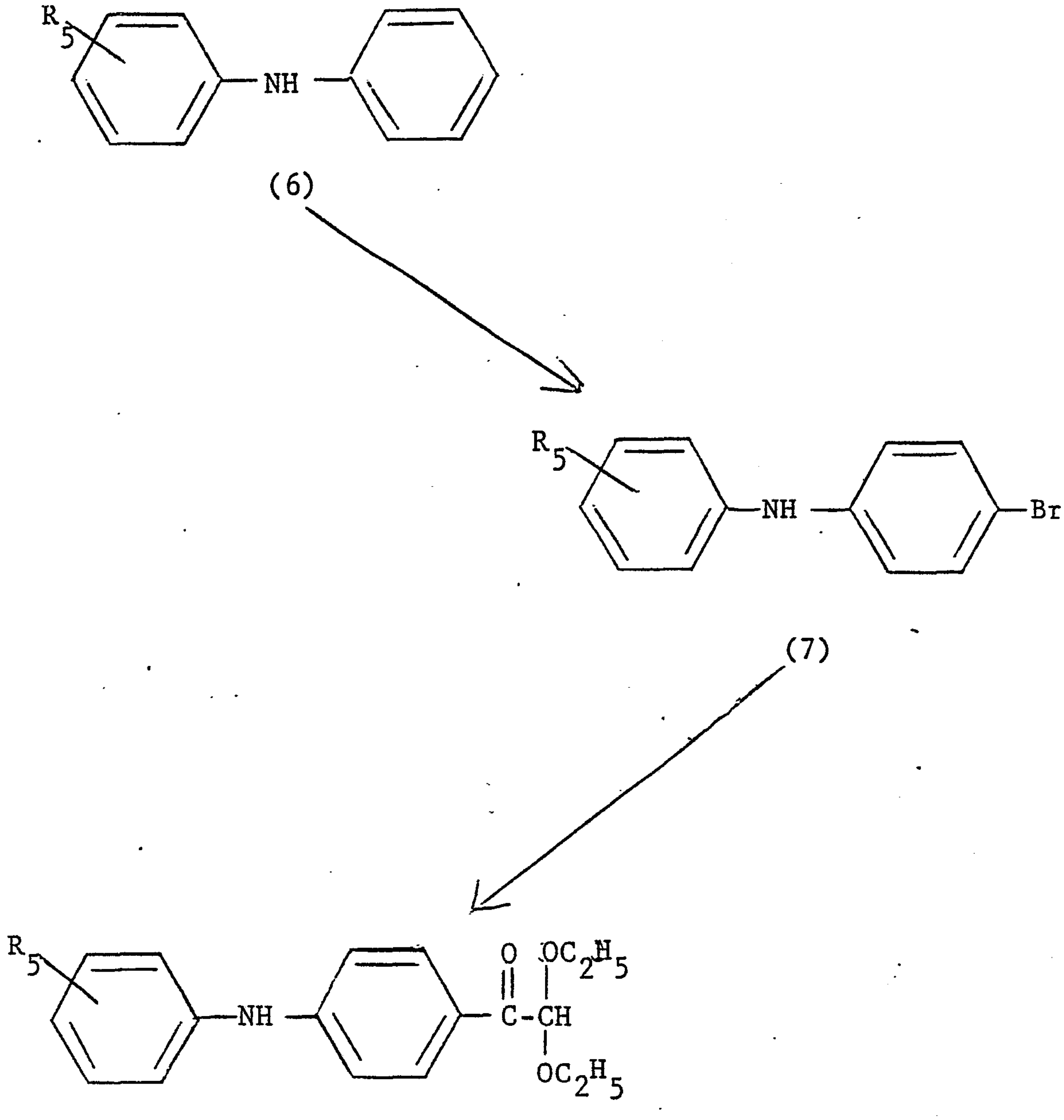

(6)

(7)

(8)

is treated with aqueous hydrobromic acid in dimethylsulfoxide for 4-10 hours, then poured into a mixture of ice and hydrochloric acid and extracted with methylene chloride. The extracts are concentrated to yield (7), which is then dissolved in ether or tetrahydrofuran in an inert atmosphere at -78° to -20°C. and treated with n-butyl lithium for 1-24 hours at -78° to 25°C. The lithium reagent thus obtained is treated with 1-(diethoxyacetyl)piperidine. This mixture is

quenched with acetic acid in ether solution. The ether layer is separated, evaporated and purified by chromatography giving (8) where $R_5$ is as described above. This diethoxy derivative is then converted to (4) by treatment with aqueous hydrochloric acid in dioxane followed by basification in ether giving (4) where $R_5$ is as described above and A is -NH-, and after treatment with sodium bisulfate as described above, compound (5).

Other arylglyoxals of this invention may be prepared as set forth in the following reaction scheme:

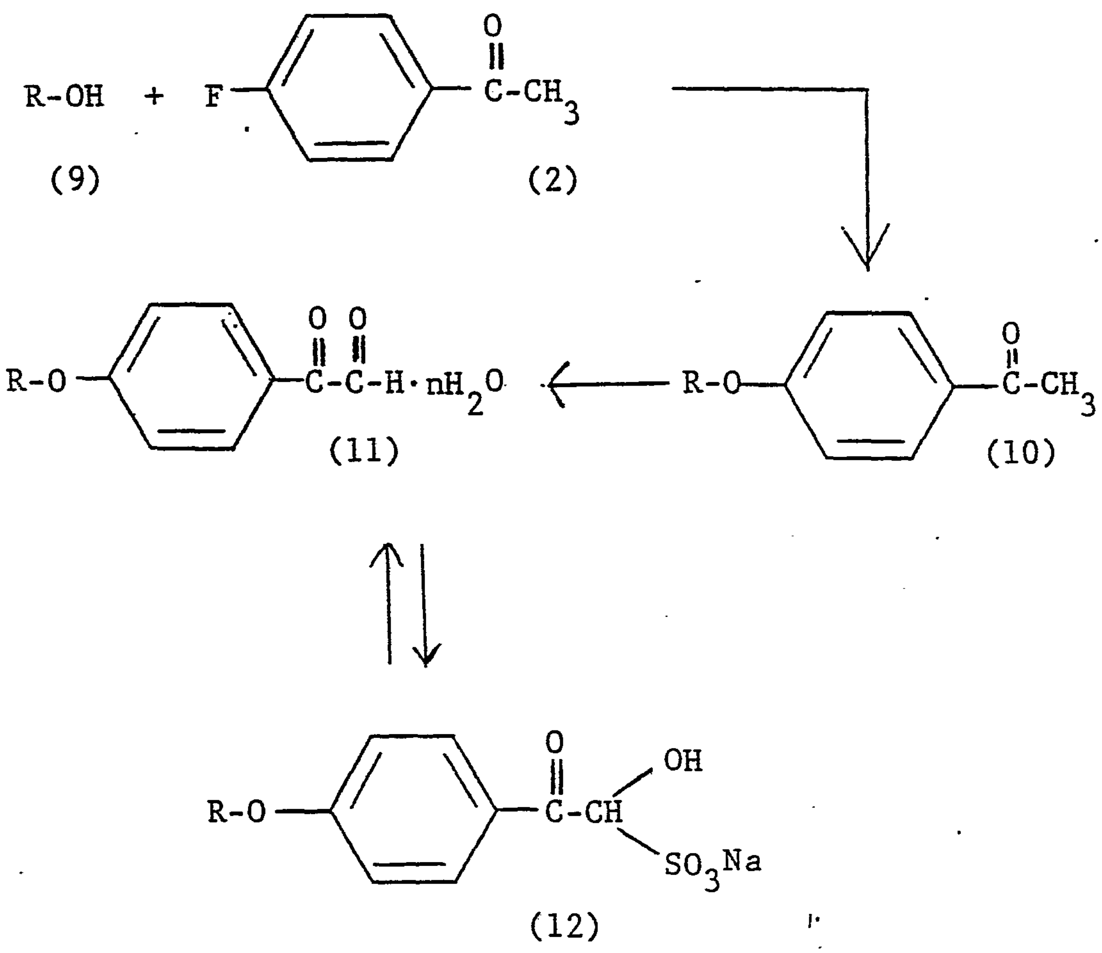

(9) (2) (11) (10) (12)

wherein R is 1-naphthyl, 4-chloro-1-naphthyl or 4-chloro-2-naphthyl. In accordance with the above reaction sequence, an appropriately substituted phenol (9) is combined with a fluoroacetaophenone (2) and treated with potassium carbonate in dimethylacetamide under an inert atmosphere at reflux for 8-48 hours, then cooled, poured into ice and extracted with ether. The extract is evaporated and distilled, giving the acetophenone (10). The acetophenone (10) is then dissolved in dimethylsulfoxide and treated with aqueous hydrobromic acid at 45-65°C. for 18-48 hours, poured into ice and extracted with ethyl acetate. The extract, which contains (11) wherein n may vary from almost zero to one or more, is concentrated, dissolved in a mixture of ethanol and water at 5-70°C. and treated with sodium metabisulfite at the boiling point for 5 minutes, then cooled under argon at 0°C., giving (12). The sodium bisulfite derivative (12) is then suspended in water at 40-60°C., acidified, heated at 90-100°C. for 1-2 hours, cooled and extracted with ether. The ether extract is concentrated giving (11). Compounds of structure (11) are obtained in varying degrees of hydration; that is, n may vary from almost zero to one or more.

Alternatively, as set forth in the following reaction sequence wherein R is as defined above; the acetophenone (10) is treated with selenium dioxide in aqueous dioxane at reflux, under an inert atmosphere for 12-24 hours. The reaction mixture is then filtered and the filtrate evaporated, giving (11), wherein n may vary from almost zero to one or more, which may be converted to the sodium bisulfite derivative (12) by treatment with sodium metabisulfite in aqueous ethanol.

0114389

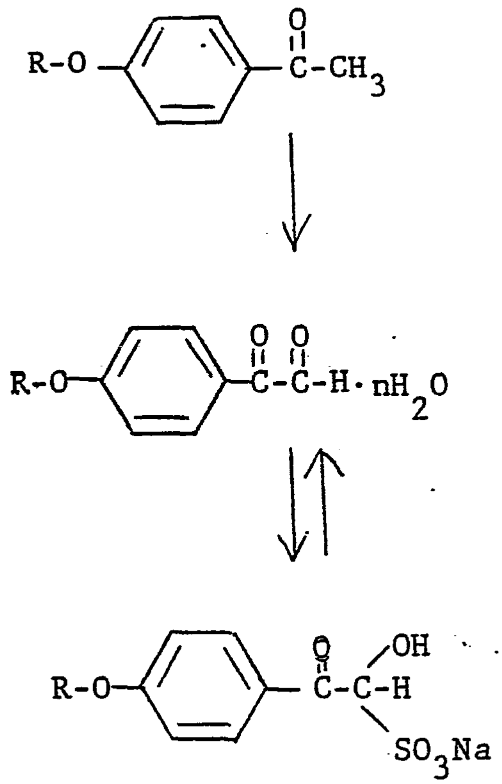

(10)

(11)

(12)

Other compounds of this invention may be prepared as set forth in the following reaction sequence:

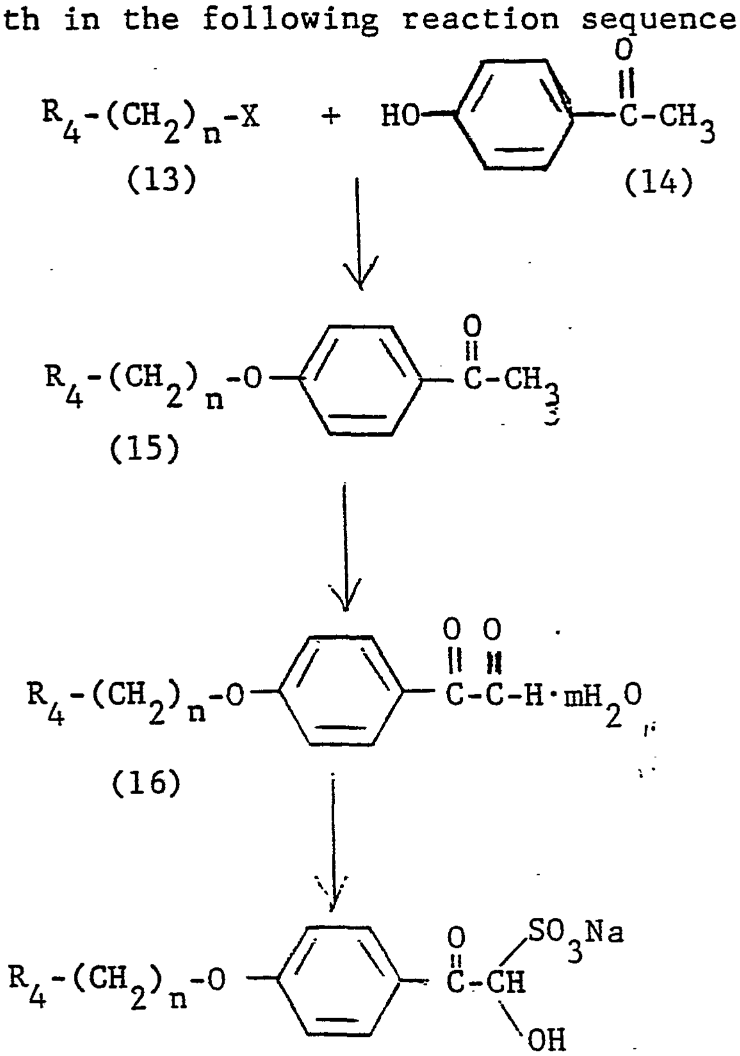

(13) (14)

(15)

(16)

(17)

In accordance with the above reaction sequence, a compound of the formula (13) where $R_4$ and n are as described hereinabove and X is chlorine or bromine, is reacted with 4-hydroxyacetophenone (14) and potassium carbonate in an organic solvent at reflux for 8-24 hours, then poured into cold water and extracted with an organic solvent giving the acetophenone (15). The acetophenone (15) is then dissolved in dimethyl sulfoxide and treated with aqueous hydrobromic acid at 45-65°C. for 18-48 hours, poured into ice and extracted with ethyl acetate. The extract containing (16) wherein m may vary from almost zero to one or more, is concentrated, dissolved in a mixture of ethanol and water at 50-70°C. and treated with sodium metabisulfite at the boiling point for 5 minutes, then cooled under argon at 0°C., giving (17). The sodium bisulfite derivative (17) is then suspended in water at 40-60°C., acidified, heated at 90-100°C., for 1-2 hours, cooled and extracted with ether. The ether extract is concentrated giving (16). Compounds of structure (16) are obtained in various degrees of hydration; that is, m may vary from almost zero to one or more. Alternatively, the acetophenone (15) is treated with selenium dioxide in aqueous dioxane at reflux, under an inert atmosphere for 12-24 hours. The reaction mixture is then filtered and the filtrate evaporated, giving (16) which may then be converted to (17) by treatment with sodium bisulfite in aqueous ethanol as described above.

The compounds of this invention were tested for their insulin-like and insulin-potentiating activity according to the following procedure. Male, Wistar strain, Royal Hart rats weighing 125-170 g. were sacrificed by decapitation. Their abdominal cavities were opened and distal or thin portions of epididymal fat pads excised, accumulated, and placed in 0.9% saline. The tissue was weighed and placed at a density of about 0.4 g./ml. in Krebs-Henseleit bicarbonate (KHB) buffer containing 5 mg. of crude bacterial collagenase per ml. [KHB is composed of 118mM sodium chloride; 4.7mM potassium chloride; 1.2mM calcium chloride; 1.2mM potassium dihydrogen phosphate; 1.2mM magnesium sulfate heptahydrate; 25mM sodium bicarbonate and

0.3mM glucose and is saturated with oxygen:carbon dioxide (95.5).] The tissues were incubated with collagenase for one hour at 37°C. with gentle agitation in a Dubnoff metabolic shaker. At the end of this digestion period the cells were washed five times with twice their volume of KHB buffer containing fatty acid free bovine serum albumin (Pentex Fraction V) at a concentration of 3%. The digest was filtered through two layers of gauze and suspended in KHB buffer with albumin to a volume ten times the initial total weight of the fat pads. Incubation of one ml. aliquots of the cell suspension was carried out in 17 x 100mm plastic Falcon tubes. Cells were incubated in the presence or absence of test compound and insulin. All tubes contained 0.15μCi D-glucose-U-$^{14}$C (specific activity >200 mC/mole).

Recrystallized porcine insulin (specific activity =25.5 U/mg.) was dissolved in 0.9% saline adjusted to pH 3 with hydrochloric acid. The insulin was added to the cells at a concentration of approximately 5 μU/ml. and control or basal cells received comparable volumes of pH 3 saline. Test compounds were dissolved in 50% dimethyl sulfoxide-50% ethanol and added to the cells at a concentration of 100 μg./ml. Control cells received comparable volumes of dimethyl sulfoxide-ethanol.

After the tubes were loaded with insulin and test compound, or other vehicles, and cell suspension, they were capped with sleeve stoppers fitted with hanging, plastic center wells. Each well contained a small section of folded filter paper. The tubes were then gassed for about one minute with oxygen:carbon dioxide (95:5) via needles inserted through the septum of the stopper. Immediately after gassing, the radioactive glucose was injected into the incubate and the tubes were placed in a

$37^oC$. metabolic shaking water bath and were incubated for one hour with agitation.

At the end of the incubation, 0.4 ml. of Hyamine hydroxide and then 0.5 ml. of 5N sulfuric acid were carefully injected into the center well and cell suspension, respectively. The acidified cell suspension was then incubated an additional 30 minutes at room temperature with gentle agitation. At the end of this carbon dioxide collection period, the center wells were dropped into vials containing 10 ml. of Dimiscint® scintillation cocktail and the radioactivity counted by liquid scintillation spectrometry.

The measurement of carbon dioxide radioactivity in counts per minute produced by these cells in the absence of both test compound and insulin is the basal level (b). Radioactivity produced in the presence of test compounds only, insulin only and both test compound and insulin are (c), (i) and (ci), respectively. Each of (c), (i) and (ci) is then expressed as a percent of the basal value: $C = \frac{c}{b}$; $I = \frac{i}{b}$; $CI = \frac{ci}{b}$. Finally, insulin-like activty (%C/I) is calculated using the formula $\%C/I = \frac{(100)\ (C-100)}{(I-100)}$; and insulin-potentiating activity (%P) is calculated using the formula

$$\%P = \frac{(100)\ (CI-C-I+100)}{(I-100)}.$$

The results of this test on representative compounds of this invention appear in Table I.

TABLE I

| Compound | %C/I | %P |
|---|---|---|
| hydroxy[p-(α,α,α-trifluoro-m-tolyloxy)benzoyl]-methanesulfonic acid, sodium salt, hemihydrate | 404 | 222 |
| α-oxo-4-[3-(trifluoromethyl)phenoxy]benzene-ethanal, hemihydrate | 1229 | 205 |
| (p-anilinobenzoyl)hydroxy-methanesulfonic acid, sodium salt | 323 | 147 |
| p-[[α-hydroxy-p-(α,α,α-trifluoro-m-tolyloxy)-phenacyl]amino]benzoic acid | 435 | 753 |
| 4-[[1-ethoxy-2-oxo-2-[4-[3-(trifluoromethyl)-phenoxy]phenyl]ethyl]amino]benzoic acid | 270 | 71 |
| α-hydroxy-β-oxo-4-[[3-(trifluoromethyl)phenyl]-thio]benzeneethanesulfonic acid, sodium salt | 195 | 275 |
| α-hydroxy-β-oxo-4-[4-(trifluoromethyl)phenoxy]-benzeneethanesulfonic acid, sodium salt, hemihydrate | 108 | 205 |
| 2,2-hydroxy-1-[4-[4-(trifluoromethyl)phenoxy]-phenyl]ethanone | 377 | 95 |
| 2,2-hydroxy-1-[4-[4-(methylthio)phenoxy]-phenyl]ethanone | 257 | 77 |
| 2,2-hydroxy-1-[4-[[3-(trifluoromethyl)phenyl]-thio]phenyl]ethanone | 816 | 0 |
| α-hydroxy-β-oxo-4-[[3-(trifluoromethyl)phenyl]-amino]benzeneethanesulfonic acid, sodium salt | 515 | 136 |
| 4-[(3-chlorophenyl)amino]-α-hydroxy-β-oxo-benzeneethanesulfonic acid, sodium salt | 370 | 165 |
| 4-nitro-α-oxo-benzeneacetaldehyde hemi-hydrate | 583 | 52 |
| 2,2-dihydroxy-1-[4-(1-piperidinyl)phenyl]-ethanone | 391 | 25 |

TABLE I (continued)

| Compound | %C/I | %P |
|---|---|---|
| [p-(4-chloro-1-naphthyloxy)benzoyl]hydroxy-methanesulfonic acid, sodium salt | 90 | 156 |
| (4-chloro-3-methnylbenzoyl)hydroxy-methane-sulfonic acid, sodium salt | 643 | 477 |
| 4-chloro-3-methyl-α-oxo-benzeneacetaldehyde hydrate | 1468 | 405 |
| (2,4-dichlorobenzoyl)hydroxy-methanesulfonic acid, sodium salt | 134 | 223 |
| (6-chloro-3-methylbenzoyl)hydroxy-methanesul-fonic acid, sodium salt | 316 | 202 |
| 4'-chloro-2,2-dihydroxy-3'-methylacetophenone | 1467 | 404 |
| 3'-5'-dichloro-2,2-dihydroxy-acetophenone | 390 | 70 |
| α-hydroxy-β-oxo-3-phenoxy-benzeneethanesul-fonic acid, sodium salt | 152 | 70 |
| 4-(4-chloro-2-naphthyloxy)-α-oxo-benzene-acetaldehyde, hemihydrate | 311 | 286 |
| 3-(3,5-dichlorophenoxy)-α-hydroxy-β-oxo-ben-zeneethanesulfonic acid, sodium salt | 122 | 152 |
| 3-(3,4-dichlorophenoxy)-α-hydroxy-β-oxo-ben-zeneethanesulfonic acid, sodium salt | 129 | 154 |
| 4-[[2-[4-(4-chloro-1-naphthyloxy)phenyl]-1-ethoxy-2-oxoethyl]amino]-benzoic acid | 250 | 305 |
| α-hydroxy-4-(1-naphthyloxy)-β-oxo-benzene-ethanesulfonic acid, sodium salt | 114 | 150 |
| 2,2-dihydroxy-1-[4-(1-naphthyloxy)phenyl]-ethanone | 481 | 96 |
| 4-[[1-ethoxy-2-(3-phenoxyphenyl)-2-oxoethyl]-amino]-benzoic acid | 75 | 161 |
| 3-chloro-α-hydroxy-4-methyl-β-oxo-benzene ethanesulfonic acid, sodium salt | 517 | 217 |
| 3-chloro-4-methyl-α-oxo-benzeneacetaldehyde hydrate | 935 | 178 |

TABLE I (continued)

| Compound | %C/I | %P |
|---|---|---|
| 4-[2-(4-fluorophenyl)ethoxy]-α-hydroxy-β-oxo-benzeneethanesulfonic acid, sodium salt | 471 | 0 |
| 4-[(3-fluorophenyl)methoxy]-α-hydroxy-β-oxo-benzeneethanesulfonic acid, sodium salt | 370 | 0 |
| 4-[(4-fluorophenyl)methoxy]-α-oxo-benzene-acetaldehyde hemihydrate | 248 | 0 |
| 4-[(2-fluorophenyl)methoxy]-α-hydroxy-β-oxo-benzeneethanesulfonic acid, sodium salt | 293 | 0 |
| α-hydroxy-β-oxo-4-[2-[3-(trifluoromethyl)-phenyl]ethoxy]-benzeneethanesulfonic acid, sodium salt | 350 | 0 |
| α-hydroxy-β-oxo-4-[[4-(trifluoromethyl)phenyl]-methoxy]-benzeneethanesulfonic acid, sodium salt | 496 | 175 |
| 4-[(3-chlorophenyl)methoxy]-α-hydroxy-β-oxo-benzeneethanesulfonic acid, sodium salt | 288 | 138 |
| α-oxo-4-[(pentafluorophenyl)methoxy]-benzene-acetaldehyde hydrate | 1076 | 259 |
| 4-[(4-chlorophenyl)methoxy]-α-oxo-benzene-acetaldehyde hydrate | 692 | 33 |
| α-oxo-4-[(3-phenoxyphenyl)methoxy]-benzene-acetaldehyde hemihydrate | 277 | 152 |
| 4-[(4-fluorophenyl)methoxy]-α-hydroxy-β-oxo-benzeneethanesulfonic acid, sodium salt | 406 | 277 |
| 4-(cyclobutylmethoxy)-α-hydroxy-β-oxo-benzene-ethanesulfonic acid, sodium salt | 296 | 274 |
| 4-[(4-methoxyphenyl)methoxy]-α-oxo-benzene-acetaldehyde | 270 | 83 |
| 4-[(4-chlorophenyl)methoxy]-α-hydroxy-β-oxo-benzeneethanesulfonic acid, sodium salt | 638 | 355 |

TABLE I (continued)

| Compound | %C/I | %P |
|---|---|---|
| α-hydroxy-β-oxo-4-[(pentafluorophenyl)methoxy]-benzeneethanesulfonic acid, sodium salt | 668 | 188 |
| α-hydroxy-β-oxo-[(3-phenoxyphenyl)methoxy]-benzeneethanesulfonic acid, sodium salt | 83 | 139 |
| α-hydroxy-β-oxo-4-[(4-methylphenyl)methoxy]-benzeneethanesulfonic acid, sodium salt | 73 | 77 |
| α-hydroxy-4-[(4-methoxyphenyl)methoxy]-β-oxo-benzeneethanesulfonic acid, sodium salt | 297 | 146 |
| 4-[(4-methylphenyl)methoxy]-α-oxo-benzene-acetaldehyde hydrate | 591 | 148 |
| [p-(cyclohexylmethoxy)phenyl]glyoxal hemihydrate | 229 | 170 |
| (α-cyclohexyl-p-anisoyl)hydroxy-methanesulfonic acid, sodium salt | 496 | 267 |
| α-hydroxy-β-oxo-4-(2-phenylethoxy)-benzene-ethanesulfonic acid, sodium salt | 466 | 197 |
| 4-[[2-[4-(cyclohexylmethoxy)phenyl]-2-oxo-1-hydroxyethyl]amino]-benzoic acid | 284 | 0 |
| 4-[[3-(trifluoromethyl)phenyl]methoxy]-α-oxo-benzeneacetaldehyde hemihydrate | 419 | 0 |
| 4-[(3-fluorophenyl)methoxy]-α-oxo-benzene-acetaldehyde hemihydrate | 114 | 55 |
| α-hydroxy-β-oxo-4-[[3-(trifluoromethyl)phenyl]-methoxy]benzeneethanesulfonic acid, sodium salt | 476 | 111 |
| α-oxo-4-[[4-(trifluoromethyl)phenyl]methoxy]-benzeneacetaldehyde hydrate | 359 | 46 |
| 4-[(3-chlorophenyl)methoxy]-α-oxo-benzene-acetaldehyde hydrate | 441 | 34 |

TABLE I (continued)

| Compound | %C/I | %P |
|---|---|---|
| α-hydroxy-β-oxo-4-[3-(trifluoromethyl)phenoxy]-benzeneethanesulronic acid, sodium salt | 124 | 187 |
| α-hydroxy-β-oxo-4-[3,5-bis(trifluoromethyl)-phenoxy]benzeneethanesulfonic acid, sodium salt | 222 | 277 |
| 2,2-dihydroxy-1-[4-(phenylamino)phenyl]ethanone | 267 | 0 |
| α-hydroxy-β-oxo-4-[[4-(trifluoromethyl)phenyl]-amino]benzeneethanesulfonic acid, sodium salt | 158 | 0 |
| α-hydroxy-4-[(3-methylphenyl)amino]-β-oxo-benzeneethanesulfonic acid, sodium salt | 507 | 228 |
| α-hydroxy-4-(methylphenylamino)-β-oxo-benzene-ethanesulfonic acid, sodium salt | 162 | 105 |
| α-oxo-4-[3-(trifluoromethyl)phenoxy]benzene-ethanal, monohydrate | | |

When the compounds are employed for the above utility, they may be combined with one or more pharmaceutically acceptable carriers, e.g., solvents, diluents and the like, and may be administered orally in such forms as tablets, capsules, dispersible powders, granules, or suspenisons containing, for example, from about 0.5 to 5% of suspending agent, syrups containing, for example, from about 10 to 50% of sugar, and elixirs containing, for example, from about 20 to 50% ethanol, and the like, or parenterally in the form of sterile injectable solutions or suspensions containing from about 0.5 to 5% suspending agent in an isotonic medium. These pharmaceutical preparations may contain, for example, from about 0.5% up to about 90% of the active ingredient in combination with the carrier, more usually between 5% and 60% by weight.

The effective dosage of active ingredient employed may vary depending on the particular compound

employed, the mode of administration and the severity of the condition being treated. However, in general, satisfactory results are obtained when the compounds of the invention are administered at a daily dosage of from about 5 milligrams to about 100 milligrams per kilogram of animal body weight, preferably given in divided doses two to four times a day, or in sustained release form. For most large mammals, the total daily dosage is from about 500 milligrams to about 5,000 milligrams preferably from about 350 milligrams to 3,500 milligrams. Dosage forms suitable for internal use comprise from about 25 to 500 milligrams of the active compound in intimate admixture with a solid or liquid pharmaceutically acceptable carrier. This dosage regimen may be adjusted to provide the optimal therapeutic response. For example, several divided doses may be administered daily or the dose may be proportionally reduced as indicated by the exigencies of the therapeutic situation. A decided practical advantage is that these active compounds may be administered orally as well as by intravenous, intramuscular, or subcutaneous routes if necessary. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, e.g., vitamin E, ascorbic acid, BHT and BHA.

The preferred pharmaceutical compositions from the stand-point of ease of preparation and administration are solid composition, particularly tablets and hard-filled or liquid-filled capsules. Oral administration of the compounds is preferred.

These active compounds may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

The invention will be more fully described in conjunction with the following specific examples which are not to be construed as limiting the scope of the invention.

## Example 1

### 4'-[3-(Trifluoromethyl)phenoxy]acetophenone

A stirred mixture of 90.6 g of 3-(trifluoromethyl)phenol, 55.2 g of 4-fluoroacetophenone and 400 ml of dry dimethylacetamide was treated under argon with 71.9 g of anhydrous potassium carbonate in portions. The mixture was stirred and refluxed at 155-160°C on an oil bath for 18 hours. The mixture was cooled, poured into 1.5 Kg of ice and extracted with three portions of ether. The extracts were combined, washed successively with water, three 100 ml. portions of cold 1 N sodium hydroxide, water

and then brine, dried and evaporated to an oil. This oil was distilled at 105-120°C., 0.15-0.20 mm Hg, giving the desired intermediate as an oil, bp 130-142°C. (0.2 mm).

Using other phenol and thiophenol derivatives in the procedure of Example 1, the intermediates of Examples 2-5 (Table II) were obtained.

TABLE II

| Example | Intermediate | Physical Constant |
|---|---|---|
| 2 | 4'-[3-(Trifluoromethyl)phenylthio]-acetophenone | bp 110-115°C (0.12 mm Hg) |
| 3 | 4-[4-(trifluoromethyl)phenoxy]-acetophenone | bp 100-110°C (0.08 mm Hg) |
| 4 | 4-[4-(methylthio)phenoxy]-acetophenone | mp 83-84°C |
| 5 | 4-[3,5-bis(trifluoromethyl)-phenoxy]acetophenone | bp 80-90°C (0.06 mm Hg) |

Example 6

Hydroxy[p-(α,α,α-trifluoro-m-tolyloxy)benzoyl]-methanesulfonic acid, sodium salt

To a stirred solution of 353 g. of 4'-[3-trifluoromethyl)phenoxy]acetophenone in 2150 ml of dimethyl sulfoxide was added 430 ml of 48% aqueous hydrobromic acid during 15 minutes. The solution was stirred at 55°C for 24 hours, then poured into 6 Kg of ice and extracted with four 1000 ml portions of ethyl acetate. The extracts were combined, washed successively with water, saturated sodium bicarbonate and brine, dried and concentrated *in vacuo* to an oil. This oil was dissolved in 2520 ml of ethanol and 840 ml of water at 60°C with stirring and treated with

133 g. of sodium metabisulfite. The mixture was stirred at the boiling point for 5 minutes and then cooled under argon to 0°C. The resulting solid was collected, washed with ethanol and ether and dried, giving the desired product as a white solid, m.p. 190-210°C. (dec.).

Example 7

α-Oxo-4-[3-(trifluoromethyl)phenoxy]benzeneethanal, monohydrate

A 374 g. portion of hydroxy[p-(α,α,α-trifluoro-m-tolyloxy)benzoyl]methanesulfonic acid, sodium salt was suspended in 3485 ml. of water and the mixture was stirred at 50°C. while 316 ml. of 6 N hydrochloric acid were added during 30 minutes. The cloudy solution was warmed slowly until sulfur dioxide evolution ceased and an oil began to separate at about 75°C., then was stirred and heated at reflux for 1.5 hours. The mixture was cooled and extracted with three 1000 ml. portions of ether. The extracts were combined, washed with water, saturated sodium bicarbonate and brine, dried and concentrated to a yellow solid. This solid was recrystallized from ether:hexane (1:1) at 5°C., to yield the desired product as a white solid, m.p. 73-80°C.

Example 8

α-Oxo-4-[3-(trifluoromethyl)phenoxy]benzeneethanal, hemihydrate

A 75 g. portion of α-oxo-4-[3-(trifluoromethyl)-phenoxy]benzeneethanl, monohydrate was dissolved in 700 ml. of boiling ether. The solution was diluted with 700 ml. of hexane and then concentrated to a volume of about 1000 ml. by boiling. This solution was cooled to approximately 5° and filtered to yield the desired product as a white solid, m.p. 78-86°C.

Example 9

α-Hydroxy-α-oxo-4-[[3-(trifluoromethyl)phenyl]thio]-benzeneethanesulfonic acid, sodium salt

A mixture of 14.8 g. of 4'-[3-(trifluoromethyl)-phenylthio]acetophenone, 5.83 g. of selenium dioxide,

3 ml. of water and 50 ml. of dioxane was refluxed under argon for 18 hours. The reaction was filtered through hydrous magnesium silicate and evaporated to an oil, which was the glyoxal hydrate corresponding to the title compound.

A 19.74 g. portion of this oil was dissolved in 50 ml. of ethanol and treated with 50 ml. of saturated aqueous sodium bisulfite. A 25 ml. portion of ethanol was added, the mixture was stirred overnight and the solid recovered by filtration. This solid was boiled in water, cooled and filtered, giving the desired product as a white crystalline solid, m.p. 170°C. (dec.).

Reaction of the intermediates of Examples 1-5 by the procedures of Example 6, 7, 8, or 9 gave the products of Examples 10-15 (Table III).

TABLE III

| Example | Intermediate of Example | Product | Physical Constant |
|---|---|---|---|
| 10 | 3 | α-hydroxy-β-oxo-4-[4-(trifluoromethyl)-phenoxy]benzeneethanesulfonic acid, sodium salt | mp 210° (dec.) |
| 11 | 3 | 2,2-dihydroxy-1-[4-[4-(trifluoromethyl)-phenoxy]phenyl]ethanone | mp 78-84°C |
| 12 | 4 | 2,2-dihydroxy-1-[4-[4-(methylthio)-phenoxy]phenyl]ethanone | mp 103-113°C |
| 13 | 2 | 2,2-dihydroxy-1-[4-[[3-(trifluoro-methyl)phenyl]thio]phenyl]ethanone | mp 86-93°C |
| 14 | 5 | α-hydroxy-β-oxo-4-[3,5-bis(trifluoro-methyl)phenoxy]benzeneethanesulfonic acid, sodium salt | |
| 15 | 1 | α-oxo-4-[3-(trifluoromethyl)phenoxy]-benzeneethanal, hydrate | |

Example 16

2,2-Diethoxy-1-[4-(phenylamino)phenyl]ethanone

A 33.8 g portion of diphenylamine was treated with a 20% solution of hydrobromic acid in dimethylsulfoxide (46 ml of 48% hydrobromic acid in 300 ml of dimethylsulfoxide) with rapid stirring for 7 hours. The solution was then poured into a mixture of 200 g of ice and 16 ml of 5 N sodium hydroxide and then extracted into methylene chloride. The extract was washed with water, dried and evaporated to a solid, then recrystallized from petroleum ether, giving p-bromodiphenylamine.

A 2.48 g portion of p-bromodiphenylamine was stirred in 10 ml of ether under argon at -20°C, while 5.7 ml fo 2.1M n-butyllithium in hexane was added via a syringe over 5 minutes. The mixture was stirred for 24 hours with the addition of 5.7 ml of 2.1M n-butyllithium in hexane at 22 hours. The mixture was cooled in an ice bath and treated over 5 minutes at 0-10°C with a solution of 1-(diethoxyacetyl)piperidine in 10 ml of ether. The mixture was then stirred at room temperature followed by refluxing for 4 hours, then poured into a mixture of one ml of acetic acid in ether and ice. The ether layer was separated, washed with 1% aqueous acetic acid until the wash was acidic, then with water, saturated aqueous sodium bicarbonate and brine, dried and evaporated to a solid. This crude solid was dissolved in 15 ml of hexane plus a few drops of ethyl acetate and developed on a column. Fractions were eluted with hexane and then with 2:1 hexane:ethyl acetate, the latter yielding the desired intermediate.

Reaction of other substituted diphenylamines or N-methyldiphenylamines using the procedure of Example 16, gave the intermediates of Examples 17-20 (Table IV).

TABLE IV

| Example | Intermediate | Physical Constant |
|---|---|---|
| 17 | 2,2-diethoxy-1-[4-[(3-chlorophenyl)amino]phenyl]ethanone | oil |
| 18 | 2,2-diethoxy-1-[4-[[4-(trifluoromethyl)phenyl]amino]phenyl]-ethanone | mp 110-115°C |
| 19 | 2,2-diethoxy-1-[4-[(3-tolyl)amino]-phenyl]ethanone | |
| 20 | 2,2-diethoxy-1-[4-[N-phenyl-N-methylamino]phenyl]ethanone | |

## Example 21

## (p-Anilinobenzoyl)hydroxy-methanesulfonic acid, sodium salt

A stirred mixture of 2.10 g of 2,2-diethoxy-1-[4-(phenylamino]phenyl]ethanone in 70 ml of 0.7 N hydrochloric acid and 70 ml of dioxane was refluxed for 3 hours, then cooled and the dioxane removed *in vacuo*. Ether was added with stirring and the pH adjusted to 8 with 5 N sodium hydroxide. The ether extract was separated, washed with water and brine, dried and evaporated to a red gum which is the glyoxal hydrate corresponding to the title compound.

A 1.4 g portion of this gum was dissolved in 30 ml of hot ethanol and then treated with a solution of 1.08 g of sodium bisulfite in 10 ml of water. This solution was refluxed for 15 minutes, filtered while hot and the filtrate evaporated to a residue. The residue was crystallized from ether, then slurried in water, giving the desired product as a yellow solid, mp 170-185°C (dec.).

Reaction of the intermediates of Examples 16-20 by the procedure of Example 21, gave the products of Examples 22-25 (Table V).

TABLE V

| Example | Intermediate of Example | Product | Physical Constant |
|---|---|---|---|
| 22 | 17 | 4-[(3-chlorophenyl)amino]-α-hydroxy-β-oxo-benzeneethanesulfonic acid, sodium salt | mp 185-200°C (dec.) |
| 23 | 16 | 2,2-dihydroxy-1-[4-(phenylamino)-phenyl]ethanone | foam |
| 24 | 18 | α-hydroxy-β-oxo-4-[[4-(trifluoromethyl)-phenyl]amino]benzeneethanesulfonic acid, sodium salt | mp 180-195°C (dec.) |
| 25 | 19 | α-hydroxy-4-[(3-methylphenyl)amino]-β-oxo-benzeneethanesulfonic acid, sodium salt | mp 170-190°C (dec.) |
| 26 | 20 | α-hydroxy-4-(N-phenyl-N-methylamino)-β-oxo-benzeneethanesulfonic acid, sodium salt | mp 120-145°C (dec.) |

Example 27

p-[[α-Hydroxy-p-(α,α,α-trifluoro-m-tolyloxy)phenacyl]-amino]benzoic acid

A mixture of 5.62 g of 2,2-dihydroxy-4'-(α,α,α-trifluoro- m-tolyloxy)acetophenone, 2.06 g of p-aminobenzoic acid, 56 ml of tetrahydrofuran and 19 ml of water was stirred at reflux for 45 minutes, then cooled and the tetrahydrofuran evaporated. The oily residue was extracted with ether. The extract was washed with brine, dried and evaporated to a foam. This foam was stirred vigorously in 500 ml of hexane:ether (10:1), filtered, washed with the same solvent mixture and dried. This solid was mixed with 50 ml of ether, cooled to 0°C and filtered. The filtrate was stirred and treated with petroleum ether until cloudy, then cooled and the product collected, giving the desired product as a light yellow solid, mp 120-140°C.

Example 28

4-[[1-Ethoxy-2-oxo-2-[4-[3-(trifluoromethyl)phenoxy]-phenyl]ethyl]amino]benzoic acid

A mixture of 5.62 g of 2,2-dihydroxy-4'-(α,α,α-trifluoro-m-tolyloxy)acetophenone, 2.47 g of p-aminobenzoic acid and 100 ml of ethanol was refluxed for 30 minutes and then 50 ml of toluene were added. The mixture was evaporated to dryness and the refluxing with ethanol and toluene repeated twice. Then the ethanol was evaporated giving a residue which was dissolved in 150 ml of ether, 75 ml of petroleum ether were added and the mixture cooled to 0°C, giving the desired product as an off-white powder, mp 109-122°C (dec.).

Example 29

α-Hydroxy-β-oxo-4-[[3-(trifluoromethyl)phenyl]amino]-benzeneethanesulfonic acid, sodium salt

A mixture of 13.0 g of m-trifluoromethylphenyl bromide, 8.68 g of p-acetylaminoacetophenone, 14.5 g of potassium carbonate, 0.45 g of copper powder and 40 ml of decahydronaphthalene was stirred at reflux on an oil bath

at 200°C, under argon for 64 hours. The mixture was then cooled, stirred with 200 ml of acetone, filtered and the filtrate evaporated to a semi-solid. Evaporative distillation gave a semi-solid which was dissolved in 150 ml of ethanol and 150 ml of concentrated hydrochloric acid and refluxed under argon for 30 minutes. The mixture was cooled, partially evaporated and partitioned between 50% saturated brine and ethyl acetate. The ethyl acetate layer was washed with water and brine, dried and evaporated to a semi-solid. This semi-solid was purified by chromatography, giving 4'-[3-(trifluoromethyl)phenylamino]-acetophenone which was then converted to the desired product by the procedure of Example 9, giving the product as a light amber solid, m.p. 180-195°C. (dec.).

## Example 30

### α-Hydroxy-β-oxo-3-[3-(trifluoromethyl)phenoxy]-benzeneethanesulfonic acid, sodium salt

To a stirred solution of 3.10 g of methyl methyl thiomethylsulfoxide in 20 ml of tetrahydrofuran under argon in an ice bath was added 10.4 ml. of 2.4 M n-butyllithium in hexane via a syringe during 15 minutes, maintaining the temperature at 10-15°C. The mixture was stirred at 10°C for 10 minutes, then recooled and a solution of 5.32 g of m-[m-(trifluoromethyl)phenoxy]benzaldehyde in 10 ml of tetrahydrofuran was added during 10 minutes, maintaining the temperature at <10°C. The resulting solution was stirred at 0-5°C for one hour, then the tetrahydrofuran was evaporated. The resulting yellow foam was added to dimethyl sulfoxide and cooled in an ice bath while aqueous hydrobromic acid was added. The reaction was then heated at 50°C overnight, then poured into ice and extracted with ethyl acetate. The extract was washed with water, saturated sodium bicarbonate and brine, dried and evaporated, giving 2,2-dihydroxy-3'-(α,α,α-trifluoro-m-tolyloxy)acetophenone as a yellow oil.

The above oil, 1.2 g of sodium bisulfite, 54 ml of ethanol and 18 ml of water were combined, heated on a steam bath for 10 minutes, then stirred at room temperature overnight. The solvent was evaporated and the residue stirred vigorously with acetone and the resulting solid collected. This solid was stirred vigorously with ethyl acetate, warmed and the solid collected, giving the desired product as a white solid.

Example 31

4-(Phenethyloxy)acetophenone

A stirred mixture of 18.5 g. of phenethylbromide, 20.4 g. of p-hydroacetophenone , 17.3 g. of anhydrous potassium carbonate and 150 ml. of acetone was refluxed under argon for 22 hours. The reaction was then cooled and partitioned between ether and water. The ether layer was washed with water and then extracted with two 100 ml. portions of cold 1N sodium hydroxide. These extracts were combined, washed with water and brine, dried and evaporated to an oil. The oil was purified by chromatography, giving the desired intermediate as a light yellow low oil.

Reaction of other substituted alkyl halides as hereinabove described with p-hydroxyacetophenone according to the procedure of this example gave the acetophenone intermediates of Examples 32-46, listed in Table VI.

TABLE VI

| Example | Intermediate | Physical Constant |
|---|---|---|
| 32 | 4'-(cyclohexylmethoxy)acetophenone | mp 42-43°C |
| 33 | 1-[4-[[3-(trifluoromethyl)phenyl]-methoxy]phenyl]ethanone | mp 86-87.5°C |
| 34 | 1-[4-[(3-fluorophenyl)methoxy]-phenyl]ethanone | mp 82-84°C |
| 35 | 1-[4-[[4-(trifluoromethyl)phenyl]-methoxy]phenyl]ethanone | mp 75-77°C |
| 35 | 1-[4-[(3-chlorophenyl)methoxy]-phenyl]ethanone | mp 75-77°C |
| 37 | 1-[4-[2-(4-fluorophenyl)ethoxy]-phenyl]ethanone | mp 50-52°C |
| 38 | 1-[4-[(4-fluorophenyl)methoxy]-phenyl]ethanone | mp 71-73°C |
| 39 | 1-[4-[2-[3-(trifluoromethyl)-phenyl]ethoxy]phenyl]ethanone | yellow oil |
| 40 | 1-[4-[(pentafluorophenyl)methoxy]-phenyl]ethanone | mp 87-90°C |
| 41 | 1-[4-[(4-chlorophenyl)methoxy]-phenyl]ethanone | mp 89-91°C |
| 42 | 1-[4-[(3-phenoxyphenyl)methoxy]-phenyl]ethanone | mp 54-57°C |

TABLE VI (continued)

| Example | Intermediate | Physical Constant |
| --- | --- | --- |
| 43 | 1-[4-(cyclobutylmethoxy)phenyl] ethanone | bp 100-110°C (0.1 mm Hg) |
| 44 | 1-[4-[(4-methoxyphenyl)methoxy]-phenyl]ethanone | mp 127-129°C |
| 45 | 1-[4-[(4-methylphenyl)methoxy]-phenyl]ethanone | mp 102-104°C |
| 46 | 1-[4-[(2-fluorophenyl)methoxy]-phenyl]ethanone | mp 88-89°C |

## Example 47

### α-Hydroxy-β-oxo-4-(2-phenylethoxy)-benzeneethanesulfonic acid, sodium salt

To a solution of 6.3 g. of 4'-(phenethyloxy)-acetophenone in 44 ml. of dimethyl sulfoxide was slowly added 8.84 ml. of 48% aqueous hydrobromic acid. The mixture was stirred at 55°C. for 16 hours, then poured into ice water and extracted with ethyl acetate. The extract was washed with water, then aqueous sodium bicarbonate and finally brine, dried and evaporated, giving 7.0 g. of an amber oil which is the glyoxal hydrate corresponding to the title compound.

This oil was dissolved in 90 ml. of warm ethanol, stirred, and treated with a solution of 2.85 g. of sodium bisulfite in 45 ml. of water. This mixture was refluxed for 15 minutes, filtered while hot, concentrated to 2/3 of its original volume and then cooled to 0°C. The solid was collected, washed with cold ethanol:water (3:1), ethanol, ether, and dried, giving the desired product as

a white solid, m.p. 165-180°C. (dec.).

Following the procedure of this example but using the acetophenones of Examples 31-46, the products of Examples 48-69 were obtained as indicated in Table VII.

TABLE VII

| Example | Intermediate of Example | Product | Physical Constant |
|---|---|---|---|
| 48 | 3 | 4-[[3-(trifluoromethyl)phenyl]methoxy]--α-oxo-benzeneacetaldehyde hemihydrate | mp 86-95°C |
| 49 | 4 | 4-[(3-fluorophenyl)methoxy]-α-oxo-benzeneacetaldehyde hemihydrate | mp 114-116°C |
| 50 | 3 | α-hydroxy-β-oxo-4-[[3-(trifluoromethyl)-phenyl]methoxy]-benzeneethanesulfonic acid, sodium salt | mp 203°C |
| 51 | 5 | α-oxo-4-[[4-(trifluoromethyl)phenyl]-methoxy]-benzeneacetaldehyde hydrate | mp 115-120°C |
| 52 | 6 | 4-[(3-chlorophenyl)methoxy]-α-oxo-benzeneacetaldehyde hemihydrate | mp 107-112°C |
| 53 | 7 | 4-[2-(4-fluorophenyl)-ethoxy]-α-hydroxy-β-oxo-benzeneethanesulfonic acid, sodium salt | mp 160-185°C (dec.) |

TABLE VII (continued)

| Example | Intermediate of Example | Product | Physical Constant |
|---|---|---|---|
| 54 | 4 | 4-[(3-fluorophenyl)methoxy]-α-hydroxy-β-oxo-benzeneethanesulfonic acid sodium salt | mp 345-350°C |
| 55 | 8 | 4-[(4-fluorophenyl)methoxy]-α-oxo-benzeneacetaldehyde hemihydrate | mp 107-113°C |
| 56 | 16 | 4-[(2-fluorophenyl)methoxy]-α-hydroxy-β-oxo-benzeneethanesulfonic acid, sodium salt | mp 197°C |
| 56 | 9 | α-hydroxy-β-oxo-4-[2-[3-(trifluoromethyl)phenyl]ethoxy]-benzeneethanesulfonic acid, sodium salt | mp 140-160°C (dec.) |
| 57 | 5 | α-hydroxy-β-oxo-4-[[4-(trifluoromethyl)-phenyl]methoxy]-benzeneethanesulfonic acid, sodium salt | mp 180°C |
| 58 | 6 | 4-[(3-chlorophenyl)methoxy]-α-hydroxy-β-oxo-benzeneethanesulfonic acid, sodium salt | mp 190°C |

TABLE VII (continued)

| Example | Intermediate of Example | Product | Physical Constant |
|---|---|---|---|
| 60 | 10 | α-oxo-4-[(pentafluorophenyl)methoxy]-benzeneacetaldehyde hydrate | mp 82-104°C (dec.) |
| 61 | 11 | 4-[(4-chlorophenyl)methoxy]-α-oxo-benzeneacetaldehyde hydrate | mp 115-124°C |
| 62 | 2 | α-cyclohexyl-p-anisoyl)hydroxy-methane-sulfonic acid, sodium salt | mp 180°C |
| 63 | 12 | α-oxo-4-[(3-phenoxyphenyl)methoxy]-benzeneacetaldehyde hemihydrate | mp 47-58°C |
| 64 | 8 | 4-[(4-fluorophenyl)methoxy]-α-hydroxy-β-oxo-benzeneethanesulfonic acid, sodium salt | mp 320°C (dec.) |
| 65 | 13 | 4-(cyclobutylmethoxy)-α-hydroxy-β-oxo-benzeneethanesulfonic acid, sodium salt | mp 180°C |

TABLE VII (continued)

| Example | Intermediate of Example | Product | Physical Constant |
|---|---|---|---|
| 66 | 11 | 4-[(4-chlorophenyl)methoxy]-α-hydroxy-β-oxo-benzeneethanesulfonic acid, sodium salt | mp 205°C |
| 67 | 10 | α-hydroxy-β-oxo-4-[(pentafluorophenyl)-methoxy]-benzeneethanesulfonic acid, sodium salt | mp 250°C |
| 68 | 12 | α-hydroxy-β-oxo-[(3-phenoxyphenyl)-methoxy]-benzeneethanesulfonic acid, sodium salt | mp 190°C |
| 69 | 15 | α-hydroxy-β-oxo-4-[(4-methylphenyl)-methoxy]-benzeneethanesulfonic acid, sodium salt | mp 220°C |

## Example 70

## [p-(Cyclohexylmethoxy)phenyl]glyoxal hemihydrate

A mixture of 12.0 g of 4'-(cyclohexylmethoxy)-acetophenone, 5.8 g of selenium dioxide, 1.4 ml of water and 70 ml of p-dioxane was refluxed with stirring at 120°C for 18.5 hours, cooled and filtered through diatomaceous earth, washing with p-dioxane. The combined filtrate and wash was poured into 200 ml of water cooled to 8°C. The yellow solid was collected and dried. An 8.1 g portion of sodium bisulfite was dissolved in 500 ml of water. To this was added a solution of the above solid in a mixture of 300 ml of ethanol and 100 ml of p-dioxane. The resulting mixture was filtered and the filtrate stored at 0°C for 48 hours. The resulting solid was collected, washed twice with water, p-dioxane and absolute ethanol and then dried. This solid was added to 535 ml of 0.5 N hydrochloric acid and heated with stirring at 75°C for 8 minutes and then cooled to 10°C. The solid was collected, dissolved in hot p-dioxane, filtered and 20 ml of water added to the filtrate. The filtrate was cooled to 0°C, giving the desired product as a white solid, m.p. 118-121°C.

## Example 71

## 4-[(4-Methoxyphenyl)methoxy]-α-oxo-benzeneacetaldehyde hydrate

A 10.0 g portion of 1-[4-[(4-methoxyphenyl)-methoxy]phenyl]ethanone was dissolved in 50 ml of hot p-dioxane. A 2.0 ml portion of water and 4.4 g of selenium dioxide were added and the mixture was stirred at reflux for 67 hours, then cooled and filtered through diatomaceous earth. The filtrate was poured into 300 ml of water and the solid collected. This solid was extracted with eight 100 ml portions of ethyl acetate. The extracts were combined, washed with water, dried and evaporated. The residue was dissolved in 100 ml of boiling toluene, treated with charcoal and filtered

through diatomaceous earth. The filtrate was treated with 200 ml. of hexane, giving the desired product as a pale yellow solid, m.p. 127-138°C.

Example 72

α-Hydroxy-4-[(4-methoxyphenyl)methoxy]-β-oxo-benzeneethanesulfonic acid, sodium salt

A 2.8 g. portion of 4-[(4-methoxyphenyl)-methoxy]-α-oxo-benzeneacetaldehyde hydrate was dissolved in 100 ml. of absolute ethanol and 100 ml. of methyl cellosolve with boiling. A solution of 1.25 g. of sodium bisulfite in 7 ml. of water was added and the mixture was heated on a steam bath for 15 minutes, then allowed to stand overnight. The solid was collected and washed with methyl cellosolve, water and then absolute ethanol, then dried, giving the desired product as a white solid, m.p. 200-205°C.

Example 73

4-[(4-Methylphenyl)methoxy]-α-oxo-benzeneacetaldehyde hydrate

A 2.8 g. portion of α-hydroxy-β-oxo-4-[(4-methylphenyl)methoxy]-benzeneethanesulfonic acid, sodium salt was combined with 25 ml. of 0.5 N hydrochloric acid, heated on a steam bath for 5 minutes and then allowed to stand for several hours. The solid was collected, slurried with 50 ml. of acetone, 25 ml. of 0.5 N hydrochloric acid were added and the mixture was refluxed for 2 hours. The solid was collected, again refluxed with acid and the solid collected, washed free of acid and dried, giving the desired product as a white solid, m.p. 125-130°C.

Example 74

4-[[2-[4-(Cyclohexylmethoxy)phenyl]-2-oxo-1-hydroxyethyl]amino]-benzoic acid

A mixture of 2.3 g. of [p-(cyclohexylmethoxy)-phenyl]glyoxal hemihydrate, 1.06 g. of p-aminobenzoic acid, 50 ml. of tetrahydrofuran and 17 ml. of water was heated at boiling for 45 minutes, stripped of tetrahydrofuran and the solid collected. This solid was recrystallized

from 200 ml. of acetonitrile, then dissolved in boiling acetone, filtered while hot and 200 ml. of water added to the hot filtrate. This mixture was cooled to 0°C. and the solid collected, giving the desired product as a pale yellow solid, m.p. 188-190°C.

## Example 75

### 4'-(4-Chloro-1-naphthyloxy)acetophenone

A mixture of 42.86 g of 4-chloro-1-naphthol, 27.6 g of p-fluoroacetophenone, 33.12 g of potassium carbonate and 200 ml of dimethylacetamide was heated at 150°C, under argon for 18 hours, then poured into ice water, stirred and extracted with ethyl acetate. The extract was washed with 0.5N sodium hydroxide, 0.5N hydrochloric acid, water and brine, dried and evaporated to a residue. This residue was boiled in hexane, then cooled and the intermediate collected to yield the product as white crystals, mp 95-99°C.

The other acetophenone starting materials used in the following examples were either commercially available or made essentially by the procedure of this example using other starting materials.

## Example 76

### 4-(Chloro-3-methylbenzoyl)hydroxy-methanesulfonic acid, sodium salt

A mixture of 16.8 g. of 4-chloro-3-methylacetophenone, 11.0 g. of selenium dioxide, 4 ml. of water and 120 ml. of dioxane was refluxed overnight, filtered through a pad of diatomaceous earth and concentrated. A 175 ml. portion of ethanol was added and the mixture was filtered through diatomaceous earth. A 9.4 g. portion of sodium bisulfite in 150 ml. of water was added and the mixture was stirred overnight. The solid was collected giving the desired product as a white powder, m.p. 195-215°C. (dec.).

## Example 77

### 4'-Chloro-2,2-dihydroxy-3'-methyl-acetophenone

A 3.0 g. portion of 4-(chloro-3-methylbenzoyl)-hydroxy-methanesulfonic acid, sodium salt was heated with hydrochloric acid for about one hour, then cooled and the solid recovered, giving the desired product as white crystals, m.p. 84-94°C. (dec.).

Following the procedures of Examples 75, 76, and 77 and using the indicated starting materials, the products of Examples 78-81, given in Table VIII, were prepared.

TABLE VIII

| Example | Starting Material | Product | Physical Constant |
|---|---|---|---|
| 78 | 3',5'-dichloroacetophenone | 3',5'-dichloro-2,2-dihydroxy-aceto-phenone | mp 75-76°C |
| 79 | 1-[4-(1-naphthyloxy)-phenyl]ethanone | α-hydroxy-4-(1-naphthyloxy)-β-oxo-benzeneethanesulfonic acid, sodium salt | Beige Solid |
| 80 | 4'-(4-chloro-1-naphthyl-oxy)acetophenone | [p-(4-chloro-1-naphthyloxy)benzoyl]-hydroxy-methanesulfonic acid, sodium salt | mp 160-180°C (dec.) |
| 81 | [p-(4-chloro-1-naphthyl-oxy)benzoyl]hydroxymeth-anesulfonic acid, sodium salt | 4-(4-chloro-2-naphthyloxy)-α-oxo-benzeneacetaldehyde, hemihydrate | mp 113-119°C |

## Example 82

## 2,2-Dihydroxy-1-[4-(1-naphthyloxy)phenyl]ethanone

A mixture of 2.62 g. of 1-[4-(1-naphthyloxy)-phenyl]ethanone, 4.54 ml of 48% hydrobromic acid and 20 ml of dimethylsulfoxide was heated at 55°C overnight and then poured into ice water and extracted with ethyl acetate. The extract was washed with water and brine, dried and evaporated to a foam. This foam was purified by chromatography and the resulting oil crystallized from cold methylene chloride, giving the desired product as a white solid, mp 93-96°C.

Following the procedure of this example but using 3'-chloro-4'-methylacetophenone as starting material and with the additional step conversion to the methanesulfonic acid, sodium salt, by treatment with aqueous sodium bisulfite, there was obtained 3-chloro-α-hydroxy-4-methyl-β-oxo-benzenlethane--sulfonic acid, sodium salt, m.p. 200-220°C. (dec.).

Example 83

3-(3,5-Dichlorophenoxy)-α-hydroxy-β-oxo-benzene ethanesulfonic acid, sodium salt

To a stirred solution of 9.3 g of methyl methylthio methylsulfoxide in 60 ml of tetrahydrofuran, in an ice bath under argon, was added a solution of 31.2 ml of 2.4M n-butyllithium in hexane, dropwise via a syringe, maintaining the temperature at 10-15$^{o}$C. The solution was stirred 15 minutes, then a solution of 16.02 g of m-(3,5-dichlorophenoxy)benzaldehyde in 30 ml of tetrahydrofuran was added, maintaining the temperature at 0-10$^{o}$C. The ice bath was removed, the mixture was stirred for one hour and the tetrahydrofuran evaporated, giving a foam. This foam was dissolved in 120 ml of dimethylsulfoxide and cooled in ice while adding 34.2 ml of 48% hydrobromic acid. The reaction was gradually heated to 50$^{o}$C overnight, then poured into cold water and extracted with ethyl acetate. The extract was washed with water, saturated sodium bicarbonate and brine, dried and evaporated. The solid residue was combined with 3.8 g of sodium bisulfite, 50 ml of water and 150 ml of ethanol, refluxed under argon for one hour, then stirred overnight. The solid was collected, giving the desired product as a white solid.

## Example 84

### 4-[[1-Ethoxy-2-(3-phenoxyphenyl)-2-oxoethyl]amino]-benzoic acid

A 3.96 g. portion of m-(phenxoy)benzaldehyde was converted to 2,2-dihydroxy-1-[(3-phenoxy)phenyl]ethanone by the procedure of Example 14 (omitting the sodium bisulfite conversion).

A 5.1 g. portion of 2,2-dihydroxy-1-[(3-phenxoy)-phenyl]ethanone and 2.74 g. of p-aminobenzoic acid in 40 ml. of toluene was refluxed under argon for one hour, then cooled and evaporated to dryness. The residue was added to 40 ml. of ethanol and refluxed for 30 minutes, then cooled to 0°C., giving the desired product, m.p. 117-127°C. (dec.).

## Example 85

### 4-[[2-[4-[(4-Chloro-1-naphthyl)oxy]phenyl]-1-ethoxy-2-oxoethyl]amino]benzoic acid

A mixture of 4.2 g. of 2,2-dihydroxy-4-(4-chloro--1-naphthyloxy)-α-oxo-benzeneacetaldehyde hemihydrate, 1.76 g. of p-aminobenzoic acid and 30 ml. of absolute ethanal was refluxed overnight under argon. The solid was collected, boiled in ethanol, cooled and filtered, giving the desired product as a pale yellow solid, m.p. 155-165°C.

0114389

29,028

## CLAIMS

1. The process for preparing compounds of the formula:

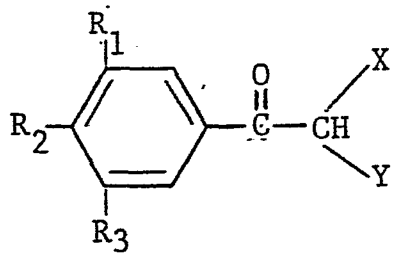

wherein $R_1$ is hydrogen, methyl or chloro; $R_2$ is hydrogen, methyl, chloro, 1-naphthlyoxy, 4-chloro-1-naphthyloxy, 4--chloro-2-naphthyloxy or moieties of the formulae:

$-O-(CH_2)_n-R_4$

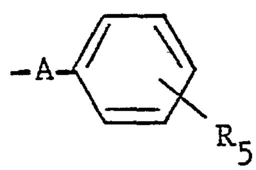

wherein n is an integer from 1 to 4 and $R_4$ is selected from the group consisting of $(C_4-C_6)$cycloalkyl, phenyl, pentafluorophenyl and monosubstituted phenyl wherein the substituent is fluoro, chloro, methyl, methoxy, trifluoromethyl or phenoxy; A is oxo (-O-), thio (-S-) or imino (-NH-) and $R_5$ represents at least one substituent selected from the group consisting of hydrogen, halogen, $(C_1-C_4)$alkylthio and trifluoromethyl; $R_3$ is hydrogen, methyl, chloro or a moiety of the formula: $-A-C_6H_4-R_5$ wherein A and $R_5$ are as hereinbefore defined; X and Y may be the same or different and are independently selected from the group consisting of hydroxy, $(C_1-C_4)$alkoxy, anilino, para-carboxyanilino, para--$(C_2-C_4)$carboalkoxyanilino and $-SO_3Q$ wherein Q is an alkali metal or alkaline earth metal and X and Y taken together represents keto (O=); and the hydrates thereof with the first proviso that $R_1$, $R_2$ and $R_3$ may not all be hydrogen, and with the second proviso that when $R_1$ and $R_3$ are hydrogen then $R_2$ may not be methyl or chloro, and with the third proviso that when n is 1

then $R_4$ may not be phenyl, and with the fourth proviso that when A is oxo or thio then $R_5$ may not be hydrogen or halogen, and with the fifth proviso that $R_2$ and $R_3$ may not both be the moiety $-A-C_6H_4-R_5$ which comprises oxidizing an acetophenone of the formula:

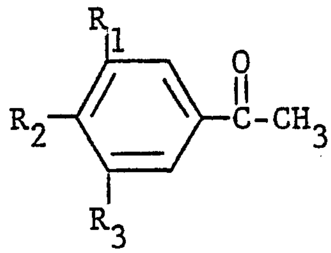

wherein $R_1$, $R_2$ and $R_3$ are as hereinbefore defined with aqueous hydrobromic acid in dimethylsulfoxide as solvent or with selenium dioxide in dioxane as solvent at $45^{\circ}$-$65^{\circ}$C. for 18-48 hours to provide an arylglyoxal of the formula:

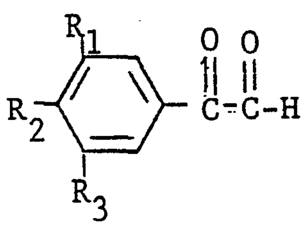

and, if desired, treating the arylglyoxal with a derivatizing agent selected from the group consisting of an alkali metal or alkaline earth metal metabisulfite, aniline, a $(C_1-C_4)$alkanol, p-aminobenzoic acid and a p-amino$(C_1-C_3)$alkylbenzoate.

2. A compound of the formula:

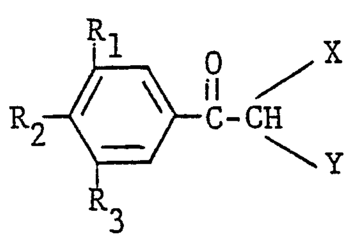

wherein $R_1$ is hydrogen, methyl or chloro; $R_2$ is hydrogen, methyl, chloro, 1-naphthyloxy, 4-chloro-1-naphthyloxy, 4--chloro-2-naphthyloxy or moieties of the formulae:

$-O-(CH_2)_n-R_4$

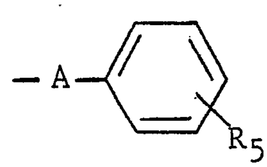

wherein n is an integer from 1 to 4 and $R_4$ is selected from the group consisting of $(C_4-C_6)$cycloalkyl, phenyl, pentafluorophenyl and monosubstituted phenyl wherein the substituent is fluoro, chloro, methyl, methoxy, trifluoromethyl or phenoxy; A is oxo (-O-), thio (-S-) or imino (-NH-) and $R_5$ represents at least one substituent selected from the group consisting of hydrogen, halogen, $(C_1-C_4)$alkylthio and trifluoromethyl; $R_3$ is hydrogen, methyl, chloro or a moiety of the formula: $-A-C_6H_4-R_5$ wherein A and $R_5$ are as hereinbefore defined; X and Y may be the same or different and are independently selected from the group consisting of hydroxy, $(C_1-C_4)$alkoxy, anilino, para-carboxyanilino, para-$(C_2-C_4)$carboalkoxyanilino and $-SO_3Q$ wherein Q is an alkali metal or alkaline earth metal and X and Y taken together represents keto (O=); and the hydrates thereof with the first proviso that $R_1$, $R_2$ and $R_3$ may not all be hydrogen, and with the second proviso that when $R_1$ and $R_3$ are hydrogen then $R_2$ may not be methyl or chloro, and with the third proviso that when n is 1 then $R_4$ may not be phenyl, and with the fourth proviso that when A is oxo or thio then $R_5$ may not be hydrogen or halogen, and with the fifth proviso that $R_2$ and $R_3$ may not both be the moiety $-A-C_6H_4-R_5$.

3. The compound according to Claim 2; hydroxy[p-(α,α,α-trifluoro-m-tolyloxy)benzoyl]methanesulfonic acid, sodium salt.

4. The compound according to Claim 2; α-oxo-4-[3-(trifluoromethyl)phenoxy]benzeneethanol.

5. The compound according to Claim 2; [p-(cyclo-hexylmethoxy)phenyl]glyoxal.

6. The compound according to Claim 2; ( -cyclo-hexyl-p-anisoyl)hydroxy-methanesulfonic acid, sodium salt.

7. The compound according to Claim 2; (4'-chloro-3-methylbenzoyl)hydroxy-methanesulfonic acid, sodium salt.

8. The compound according to Claim 2; 4'-chloro-2,2-dihydroxy-3'-methyl-acetophenone.

9. The compound according to Claim 2; [p-(4-chloro-1-naphthyloxy)benzoyl]hydroxy-methanesulfonic acid, sodium salt.

10. A pharmaceutical composition which comprises an effective antidiabetic and/or hypoglycemic amount of a compound as recited in Claim 2 in association with a pharmacologically acceptable carrier.